(19) Europäisches Patentamt
European Patent Office
Office européen des brevets

(11) EP 1 641 812 B1

(12) **EUROPEAN PATENT SPECIFICATION**

(45) Date of publication and mention of the grant of the patent:
**09.06.2010 Bulletin 2010/23**

(21) Application number: **04730317.7**

(22) Date of filing: **29.04.2004**

(51) Int Cl.:
***C07J 41/00*** *(2006.01)*

(86) International application number:
**PCT/HU2004/000030**

(87) International publication number:
**WO 2005/000867 (06.01.2005 Gazette 2005/01)**

(54) **PURE d-(17alpha)-13-ETHYL-17-HYDROXY-18,19-DINORPREGN-4-ENE-20-YNE-3-ONE-3E- AND -3Z-OXIME ISOMERS, AS WELL AS PROCESS FOR THE SYNTHESIS OF THE MIXTURE OF ISOMERS AND THE PURE ISOMERS**

d-(17alpha)-13-ETHYL-17HYDROXY-18,19-DINORPREGN-4-ENE-20-YNE-3-ONE IN FORM DES REINEN 3E- UND 3Z-OXIMES SOWIE DAS VERFAHREN ZUR HERSTELLUNG DER MISCHUNG DER ISOMERE WIE AUCH DER REINEN ISOMERE

ISOMERES PURS DE d-(17alpha)-13-ETHYL-17-HYDROXY-18,19-DINORPREGN-4-ENE-20-YNE-3-ONE-3E- ET -3Z-OXIME, ET PROCEDE POUR REALISER LA SYNTHESE DU MELANGE D'ISOMERES ET D'ISOMERES PURS

(84) Designated Contracting States:
**AT BE BG CH CY CZ DE DK EE ES FI FR GB GR HU IE IT LI LU MC NL PL PT RO SE SI SK TR**

(30) Priority: **30.06.2003 HU 0301981**

(43) Date of publication of application:
**05.04.2006 Bulletin 2006/14**

(73) Proprietor: **Richter Gedeon Nyrt.**
**1103 Budapest (HU)**

(72) Inventors:
- **TUBA, Zoltán**
  **H-1022 Budapest (HU)**
- **MAHO , Sándor**
  **H-1183 Budapest (HU)**
- **KESERU , György**
  **H-2089 Telki (HU)**
- **KOZMA, József**
  **H-1204 Budapest (HU)**
- **HORVATH, János**
  **H-1098 Budapest (HU)**
- **BALOGH, Gábor**
  **H-1183 Budapest (HU)**

(74) Representative: **HOFFMANN EITLE**
**Patent- und Rechtsanwälte**
**Arabellastrasse 4**
**81925 München (DE)**

(56) References cited:
**EP-A- 0 309 263**
**WO-A-96/40355**
**CH-A- 494 218**
**GB-A- 1 452 179**
**US-A- 4 027 019**

- **M.PATTHY ET AL.: "High-Performance Liquid chromatography and Gas-Liquid Chromatography of some Norgestrel Intermediates" J.OF CHROMATOGR., vol. 191, 1980, pages 145-154, XP009037312 cited in the application**
- **J.L.MCGUIRE: "Pharmacological and Pharmacokinetic characteristics of Norgestimate and its Metabolites" AM.J.OBSTET.GYNECOL., vol. 163, no. 6, 1990, pages 2127-2131, XP009037315 cited in the application**
- **HOUBEN-WEYL: "Methoden der Organischen Chemie Band X/4" 1968, GEORG THIEME VERLAG , STUTTGART , XP002298921 page 291**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 641 812 B1

## Description

The invention relates to plaster type pharmaceutical compositions containing the pure d-(17α)-13-ethyl-17-hydroxy-18,19-dinorpregn-4-ene-20-yne-3-one-3E-oxime isomer of formula (IA),

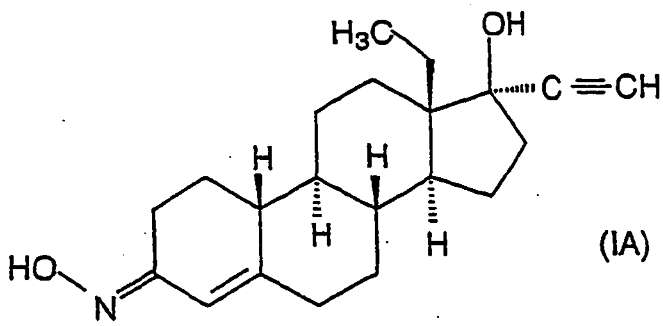

(IA)

to orally applicable pharmaceutical compositions containing the pure d-(17α)-13-ethyl-17-hydroxy-18,19-dinorpregn-4-ene-24-yne-3-one-3Z-oxime isomer of formula (IB),

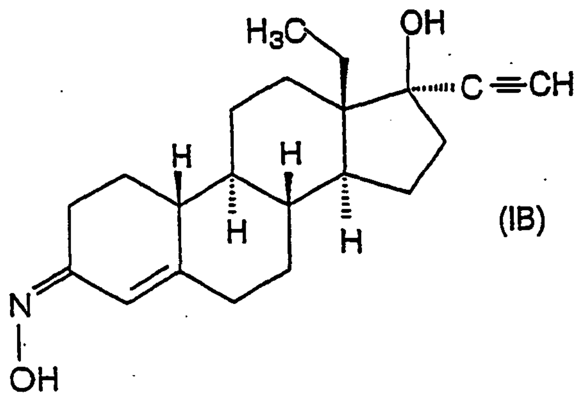

(IB)

wherein the isomers are of gestagen activity, as well as the process for the synthesis of the mixture of the above isomers and the pure isomers. The pharmaceutical compositions contain either the pure isomer of formula (IA) or the pure isomer of formula. (IB) as active ingredient as such or in combination with other active ingredients (for example an estrogen agent) together with pharmaceutical auxiliary materials commonly used in practice.

Such isomers are understood under "pure" isomers in this description, which are of the same purity as the isomers obtained in the examples.

The orally applicable pharmaceutical compositions of the present invention can preferably be tablets or dragées. The tablets can contain- beside the active ingredient(s) - the usual carriers, excipients, diluents, stabilizers, flavoring or aromatizing, as well as formulation-promoting or formulation-providing additives. The formulation of tablets can be carried out by methods conventionally used in practice. The preparation of dragées can be carried out by coating the seeds, prepared similarly to tablets, according to the usual methods.

The plaster type pharmaceutical compositions can preferably be matrix type transdermal plasters consisting of 3 layers. Their external layer is a membrane, which is impermeable for the active ingredients and other components of the matrix, consisting of PVC, polyethylene, polypropylene or polyurethane film. The matrix containing the hormone is disposed on this external layer. The matrix contains pressure sensitive adhesive component, which can be polyacrylate, polydimethylsiloxane or polyisobutylene. One of these adhesives is mixed with the active ingredients and the polyvinylpyrrolidone auxiliary material, which inhibits crystallization. Auxiliaries (enhancers), which promote the absorption of steroids through the skin, are preferably dispersed in the matrix as well. These components can be for example esters of aliphatic alcohols, such as lauryl lactate, oleic acid, etc. The so obtained dispersion is disposed on the external layer of the plaster and dried.

The matrix - until the application - is covered by the third layer of the plaster, the protective layer, which can be for example a polyethylene terephthalate film. The protective layer should be removed before the application (sticking

on the skin) of the plaster.

The synthesis and biological investigation of 3-oximino-androstene- and gonene derivatives containing sterane skeleton started in the 1960s. The application of dl-(17α)-13-ethyl-17-acyloxy-18,19-dinorpregn-4-ene-20-yne-3-one-oxime derivatives as postcoitalis contraceptives is suggested in the U.S. patent Number of 3,780,073.

Concerning, that it is very important to decrease the applied dosage of the active ingredients in the case of every active ingredient used in the therapy, which is especially true for the steroid derivatives possessing high biological activity, the aim of the research was to synthesize and investigate the biological effect of the pure optical antipodes of steroid derivatives, which had been described in the literature earlier as racemic mixtures. The synthesis of d-(17α)-13-ethyl-17-acetoxy-18,19-dinorpregn-4-ene-20-yne-3-one-oxime (norgestimate) is described in the U.S. patent Number of 4,027,019.

The biological and clinical investigation of norgestimate proved its more advantageous inhibition of fertility. The compound in combination with ethynyl-estradiol gained therapeutic application as ORTHO-CYCLEN and CILEST. The use of the optically active isomer made possible the application of the active ingredient in lower dosage than in the case of the racemic mixture.

A further progress in the research was the synthesis of 17-deacetyl-norgestimate (norelgestromine) and the pharmacological as well as clinical investigation thereof. The authors of the following publications - Am. J. Obstet. Gynecol., 166. 1969-77 (1992) and Am. J. Obstet. Gynecol., 163, 2127-31 (1990) - discovered, that the metabolites of orally applied norgestimate are the 17-deacetyl-norgestimate and the 3-keto-norgestimate (levonorgestrel acetate), as well as the d-norgestrel (levonorgestrel), which are mainly responsible for the biological activity.

The U.S. patent Number of 4,906,169 describes the use of norgestimate and d-norgestrel in combination with an estrogen component in transdermal plaster.

The patent Number of PCT WO 96/40355 discloses the use of deacetyl-norgestimate - that is one of the metabolites of norgestimate - as such or in combination with an estrogen component in transdermal plaster.

The synthesis of dl- and d-(17α)-13-ethyl-17-hydroxy-18,19-dinorpregn-4-ene-20-yne-3-one-oxime is described in the Hungarian patent Number of 165,356. The compounds are described as the intermediates of the synthesis of the racemic and optically active norgestrel, but their biological activity is not given.

The above patents gave essentially the same method for the synthesis of the oxime derivatives. They use hydroxylammonium hydrochloride as reagent, pyridine as solvent and base; the steroid is dissolved by heating in a water bath and is heated until the completion of the reaction. The product is isolated by addition of water and recrystallized. The E/Z isomer ratio of the so obtained oxime derivatives is about 60:40-64:36.

The separation of several known steroids - among them the norelgestromine - by high pressure chromatography is described in the following publication: J. Chromatogr., 392, 464-9 (1987), but only the chromatographical parameters and not the physico-chemical properties of the separated oxime isomers, which prove the structures, are given.

The examination of some intermediate of the synthesis of norgestrel by high performance liquid chromatography and gas-liquid chromatography is described in the following publication: J. Chromatogr., 191(1), 145-54. (1980). Among the above compounds there are the racemic mixture and the optically pure oxime derivatives as well, which are disclosed in the Hungarian patent Number of 165,356. But from the description it is not unambiguous, whether the optically pure or the racemic mixture of steroid oxime derivatives were examined. According to the above publication the oxime isomers were separated by normal phase analytical HPLC and their structures were elucidated. For the structure elucidation they refer to the publication of Hara and coworkers [Chem. Ind. (London), 832 (1967)], where the syn and anti oxime isomers of testosterone were separated, their structures were examined by NMR and UV spectroscopical methods; the significant difference measured in the molar absorption of the two oxime isomers at 242 nm wavelength was emphasized.

A general effort of the pharmaceutical industry is the synthesis of structurally homogeneous and stereo-chemically pure active ingredients, and their use in therapy, which means application of lower dosage of the active ingredients having clearer biological activity profile and therefore decreased side effects.

This effort led us to synthesize the E-isomer oxime of formula (IA) and Z-isomer oxime of formula (IB) of d-(17α)-13-ethyl-17-hydroxy-18,19-dinorpregn-4-ene-20-yne-3-one-oxime, which is used in therapy as a stereochemical mixture of isomers of E/Z oximes. The use of pure isomers made possible to increase the homogeneity of the biological activity profile and to take advantage of the different physical properties (for example solubility, absorption, transport) of: the individual isomers in realization of a more suitable application method in therapy.

As described above the known procedures for the synthesis of steroid compounds containing an oxime group at position 3 - the norgestimate and the 17-deacetyl-norgestimate - lead to an isomeric mixture of oximes, where the ratio of isomers is about 60:40 - 64:36 E/Z-oximes.

Surprisingly it was found, that using the process according to our invention for the oximation reaction and for the work-up procedure of the obtained mixture of oximes, either the d-(17α)-13-ethyl-17-hydroxy-18,19-dinorpregn-4-ene-20-yne-3-one-(3E)-, or -(3Z)-oxime, as well as the mixture of -(3E and Z)-oximes can be synthesized as required. The process according to our invention is the following:

a) 1 mol of d-norgestrel is reacted with 1.2-5 mol equivalent of hydroxylammonium acetate or with a hydroxylammonium salt and not more than one equivalent amount of the latter of alkali metal acetate in acetic acid containing not more than 50 mass percent of water, at 15-50 ˚C for 15-45 min and the obtained reaction mixture containing the isomeric mixture of norelgestromine

α) is diluted with about a 10-fold volume of water and the precipitated isomeric mixture is isolated to give an E/Z mixture of isomers in a ratio of about 56:44-64:36, or in given case
β) after addition of about 10-25 volume percent of water it is stirred at 10-30 ˚C for 24-72 h, in given case water is added to the reaction mixture and the precipitated product is isolated to give the (3E)-oxime isomer of formula (IA), or in given case
γ) after addition of about 10-fold volume of water the precipitated isomeric mixture is isolated and stirred in dichloromethane, the insoluble (3E)-oxime isomer of formula (IA) is filtered off, the filtrate is purified by column chromatography using silica gel as adsorbent and a mixture of apolar-polar solvents as eluent to give the (3Z)-oxime of formula (IB), or

b) a mixture of E/Z isomers of norelgestromine of any ratio

α) is stirred with hydroxylammonium acetate or with a hydroxylammonium salt and not more than one equivalent amount of the latter of alkali metal acetate in acetic acid containing not more than 50 mass percent of water, at 15-30 ˚C for 24-72 h and in given case after addition of more water the product is isolated to give the (3E)-oxime isomer of the formula (IA), or
β) is stirred in dichloromethane, the insoluble (3E)-oxime isomer of formula (IA) is filtered off, the filtrate is purified by column chromatography using silica gel as adsorbent and a mixture of apolar-polar solvents as eluent to give the (3Z)-oxime of formula (IB), or

c) the acetate group at position 17 of the 3E- or 3Z-isomer of norgestimate is hydrolyzed in alcoholic solution with equivalent amount of alkali metal hydroxide at 5-30 ˚C and the obtained product having the same configuration as the starting material is isolated to give the (3E)-oxime isomer of the formula (IA) or the (3Z)-oxime isomer of formula (IB)

and the isomers of formula (IA) and (IB) obtained according to processes a)-c) are purified by crystallization.

In process c) lithium hydroxide monohydrate is preferably used as alkali metal hydroxide and the reaction is carried out in methanol.

According to our invention, if the formation of oximes is carried out with hydroxylammonium hydrochloride and sodium acetate or hydroxylammonium acetate - prepared in advance - in glacial acetic acid or in aqueous acetic acid, then the ratio of E/Z isomers in the obtained crude isomeric mixture can be varied between 56:44 and 94:6 depending on the further treatment of the mixture. This allows for example the isolation of E-oxime isomer directly from the reaction mixture, but helps in isolating the Z-oxime isomer by column chromatography, for example from the 56:44 mixture, because this ratio can be changed to 65.5:34.5 after stirring in dichloromethane. The Z isomer can easily be isolated from this mixture for example by column chromatography.

Using process b) of our invention the pure E-isomer can be produced from any E/Z isomeric mixture or even from the pure Z-isomer by isomerisation. The detailed description of this is given in the examples.

According to process c) of our invention the pure E- or Z-oxime isomers can be obtained from the known [Journal of Chromatography, 635, 342-345 (1993)] d-(17α)-13-ethyl-17-acetoxy-18,19-dinorpregn-4-ene-20-yne-3-one-(3E)- or -(3Z)-oxime by hydrolyzing the acetoxy group at position 17. The stereochemical purity does not change under the gentle reaction conditions of hydrolysis according to our invention.

The process according to our invention can preferably be carried out the following way:

1.2-5 mol equivalent (calculated for 1 mol of d-norgestrel) of hyd-roxylammonium chloride and not more than one equivalent amount of the latter of sodium acetate are suspended in glacial acetic acid, and the obtained suspension (sodium chloride precipitates) is stirred for 30 min, then the sodium chloride is filtered off. d-Norgestrel is added to the filtrate and the reactions mixture is stirred until completion of the reaction, then diluted with water, the precipitated product is filtered off, washed with water, dried and recrystallized.

According to an other embodiment of our invention the sodium chloride is not filtered off, because after addition of water it dissolves and does not influence the yield and the quality of the product.

The E/Z ratio of the so obtained isomeric mixture of oximes is about 60:40.

Hydroxylammonium acetate prepared in advance can also be used as reagent.

If the reaction mixture is stirred for additional 24-72 h, preferably for 48 h, after consumption of the starting material - without isolating the formed oxime - and carrying out the reaction under the above reaction conditions, using glacial acetic acid or 85 % aqueous acetic acid as solvent, and the formed product is filtered off or isolated after addition of water, then the obtained isomeric mixture contains the E/Z isomers in a ratio of about 94:6.

According to an other embodiment of our invention for example a 60:40 mixture of E/Z isomers or even pure Z-isomer is suspended in acetic acid containing hydroxylammonium hydrochloride and not more than one equivalent amount of the latter of sodium acetate and the above reaction conditions are applied. In this case an isomeric mixture is obtained containing the E/Z isomers in a ratio of 90:10-96:4.

If in the first part of process a) the oximation reaction is carried out by keeping the reaction mixture homogeneous, and immediately after consumption of the starting material the reaction mixture is diluted with water and the precipitated solid product is isolated, then the isomeric ratio of the obtained mixture is 56:44 E/Z oximes. This isomeric mixture is stirred with dichloromethane in step $\gamma$) of process a). In this case the insoluble E-isomer can be filtered off, and the isomeric ratio in the filtrate can be changed for the Z-isomer (about E/Z=33:77), which helps in the isolation of Z-isomer by column chromatography.

The separation of E- and Z-isomers is preferably carried out by column chromatography using silica gel as adsorbent and starting the elution with a predominantly apolar mixture of solvents and gradually increasing the concentration of the more polar solvent. The fractions containing the same isomer are concentrated and the residue is recrystallized.

A further possibility for the synthesis of pure E- or Z-isomer is given in process c). According to this process the E/Z isomeric mixture of norgestimate is separated by known chromatographical method [J. Chromatogr., 635, 342-345 (1993)], and the acetate group at position 17 of the pure E- or Z-isomer oxime is hydrolyzed by equivalent amount of alkali metal hydroxide, preferably lithium or sodium hydroxide in alcoholic solution under gentle conditions, preferably at 5-20 °C. If the hydrolysis is carried out under these conditions the stereochemistry of the hydroxyl group of the oxime group at position 3 does not change.

According to the new process of our invention the pure E-oxime isomer can be produced on industrial scale. The separation of Z-oxime isomer can be economical, because the ratio of E/Z isomers can be varied for the Z-isomer oxime and from this mixture the Z-isomer can be isolated by column chromatography.

Moreover our new process, the pure isomers of formula (IA) and (IB) are also new, because their characteristic properties, which unambiguously prove their structures, are given in this invention.

According to the relative configuration of the hydroxyl group of the oxime group of norelgestromine there are two geometric isomers of the compound. These isomers can be separated by usual column chromatography and the Z isomer is more polar, than the E isomer. The application of isomeric mixtures of norelgestromine in transdermal plasters raise the question, that the absorption of the isomers having different polarity through the skin can be different. Next we investigated this presumption. Our investigations included the physico-chemical properties of the pure isomers as well as their in vitro pharmacokinetical study.

During the physico-chemical study we determined the solubility of isomers in water, as well as their lipophilicity by traditional and isocratical HPLC. The in vitro pharmacokinetical study included the metabolic stability, the metabolic clearance and the Caco-2 permeability of the compounds.

## Protocol for solubility measurement

The determination of equilibrium solubility of E and Z isomers of norelgestromine was carried out in distilled water. 20 mg of norelgestromine was added to 20 ml of distilled water at room temperature. The suspension was stirred continuously and samples were withdrawn from time to time. The samples were filtered and the norelgestromine content of the filtrates was determined by spectrophotometrical method. The spectrophotometrical measurements were performed on VARIAN Cary 3E spectrophotometer at room temperature.

## Protocol for lipophilicity measurement

Lipophilicity was determined by an HPLC method. The HPLC measurements were performed on Thermo Separation Product (SpectraSystem P4000 and SpectraFOCUS Forward Optical Scanning Detector) HPLC instrument. Data were analyzed by ChromQuest (ver. 2.51) software.

For reversed phase HPLC measurements Nova-Pak C18 column was used (dimension of 4 $\mu$m x 4.6 mm x 250 mm: Waters, Ireland) detection was performed at $\lambda$=280 nm and 25 °C. The flow rate of the mobile phase was 1.0 ml/min. HPLC gradient grade acetonitrile was used as organic component (Merck KGaA., Darmstadt, Germany). The retention data of the two isomers were obtained by isocratical analysis of the mobile phases containing different amount of acetonitrile. The void time (t0) was determined by injection of methanol.

Samples were dissolved in a 1:1 mixture of acetonitrile:water in a concentration of 1 mg/4 ml. The logk' values

were calculated from the mean retention time measured after two subsequent injections of 10 μl volume (log k'= log((tR - t0)/ t0)). The log k' values were represented in a function of the concentration of acetonitrile. The void time (t0) was found to be 1.49 min in this experimental arrangement.

The chromatographical hydrophobicity index (φ0) is the degree of the lipophil character of the compounds in the reversed phase HPLC measurements. By definition the φ0 parameter is that concentration of acetonitrile of the mobile phase, where log k' = 0.

**Protocol for assessment of metabolic stability and clearance**

The metabolic stability of norelgestromine E and Z isomers was examined in human liver microsomes. The 2.5 ml incubation mixture contained 6mM of Na-pyrophosphate, 5mM of $MgCl_2$, 5mM of glucose-6-phosphate, 1 U/ml of glucose 6-phosphate dehydrogenase, human liver microsomes (1mg/ml) and 5μM of norelgestromine E or Z isomers. The pH was adjusted with 100 mM of Tris-HCl buffer to pH 7.4. The reaction was started by the addition of 5mM of NADPH. 0.5 ml samples were taken at 0, 5 and 20 minutes with immediate precipitation by 0.5 ml of ice-cold methanol. 1 ml precipitated samples were centrifuged for 30 min at 1200g and 10 μl of supernatant was injected into the HPLC.

Analytical measurements were conducted using Merck-Hitachi HPLC system, with UV monitoring at 244 nm. Unchanged material was measured and intrinsic clearance ($Cl_{int}$) and metabolic bioavailability (F%) was calculated with the following equations:

$$dc/dt/c_0 = Cl_{int1}\ (ml/min \times g\ protein),$$

where dc/dt is the concentration change in a given period of time and $c_0$ is the initial concentration of the norelgestromine isomer (measured in the 0 min sample). As well as

$$Cl_{int1} \times 45 = Cl_{int2}\ (ml/min \times g\ liver)$$

and

$$EH = Cl_{int2} / Cl_{int2} + HBF,$$

where EH is hepatic extraction and HBF is the hepatic blood flow. Finally the metabolic stability:

$$F\% = (100-EH) \times 100$$

For statistical analysis Student t-test was used (Microsoft Excel). The results summarized in Table 1 are calculated as a mean of 3 parallel measurements.

**Protocol for Caco-2 permeability measurement**

Drug absorption studies were performed with Caco-2 human adenocarcinoma (epithelial) cell line monolayers as in vitro model. Passive flux characteristics of drugs across the Caco-2 monolayer have shown a correlation with human oral bioavailability.

Caco-2 cells obtained from American Type Culture collection, Rockville, MD, (ATCC) were grown at 37˚C in an atmosphere of 5 % $CO_2$ in Dulbecco's modified eagle medium supplemented with 10% heat inactivated fetal bovine serum (GIBCOBRL 11360-039) and antibiotics: penicillin 100 U/mL, and streptomycin 100 μg/mL (GIBCOBRL 15140-031).

Confluent cell monolayers grown in an incubator (at 37 ˚C with 5 %$CO_2$/95 %$O_2$ and 95% humidity) were subcultured every seven days by treatment with 0.25 % trypsin containing 1 mM EDTA.

19-23-day-old confluent monolayers of fully differentiated Caco-2 cells were used for transport studies after 6-10 passages.

EHS Cell Attachment Matrix (Promega G5971), Minimum Essential medium Eagle (MEM) with Earle salts, L-Glutamine (GIBCOBRL 41500-091) and Transwell Polycarbonate Membrane, (Costar 3401) were used.

Coated Transwells with EHS Cell Attachment Matrix (Promega G5971) and 200 000-500 000 Caco-2 cells are applied/insert. Caco-2 monolayers grown on the luminal side/apical compartment of transwells.

The E- and Z-isomers of norelgestromin were assayed at 50 μM concentration. [$^{14}$C] Mannit was used as paracellular marker (3,7x$10^4$ Bq/test chamber).

After removal of cell culture medium, 3 parallel Caco-2 cell monolayers were preincubated for each test compounds with prewarmed (37˚C) HBBS-TRIS (400 μL to the luminal side and 1.5 mL to the basolateral side/compartment) for 20 min. at 37˚C.

Change the medium followed by addition of 0.4 mL 50-100 μM working concentration of investigated and reference molecules to the luminal/apical compartment of inserts.

Measurement of absorption (luminal side to basolateral side) was made by obtaining samples from the luminal/ apical ("donor") compartment at zero time point and from the basolateral ("receiver" side) at every 15 min (3x).

The concentrations of the isomers were determined by liquid chromatography with ultraviolet (HPLC/UV) analysis. Method: gradient elution at 35˚C. Eluent A: methanol - 0.05 M, ammonium acetate= 300-200+500 μL 10% acetic acid. Eluent B: methanol. Flow: 0.50 mL/min. Detection: 240 nm. Column type: Merck Purospher C-18

Dim.:125 - 3 mm. + guard. Chrom Type: HPLC Channel:2. Peak quantitation: height; calculation method: EXT-STD.

The abluminal concentrations of the penetrated isomers are shown in Table 1.

**Table 1**

| **Parameter** | **E-isomer** | **Z-isomer** |
|---|---|---|
| solubility (followed by UV absorption) | 3.8 μg/ml | 12.1 μg/ml |
| Polarity (k', HPLC) | 1.02 | 1.46 |
| Polarity (CHI index, HPLC) | 74 | 70 |
| Metabolic stability (%) | **86.7** ± 1.67 | **91.9** ± 1.52 |
| Metabolic clearance (ml/min g liver) | **0.1815** ± 0.026 | **0.1042** ± 0.021 |
| Concentration on the abluminal side (μM in the 30th min) | 1.27 ± 0.56 | 1.98 ± 0.78 |

As the above data show the Z-isomer of norelgestromine is more soluble in water than the E-isomer. In the case of the Z-isomer the penetration across the epithelial cell layer is faster, the metabolic stability is higher as well as the clearance is minor than in the case of the E-isomer. These properties show that after oral administration the absorption of the Z-isomer is better, than that of the E-isomer, therefore its application in orally administered formulations (for example in tablets) is more advantageous.

Siddiqui and coworkers [J. Pharm. Biopharm., 17, 405 (1989)] have shown - by carrying out experiments on isolated human skin preparations - , that the lipophil steroids penetrate faster across the human epidermis, than the polar steroids, but the rate of clearance is about the same in both cases. According to our experiments of solubility and polarity the E-isomer of norelgestromine is significantly more lipophil, than the Z-isomer. According to the experiments of Siddiqui and coworkers the penetration of the less lipophilic E-isomer is faster across the epidermis, than that of the Z-isomer, therefore the application of E-isomer in transdermal plasters is more advantageous.

The beneficial effect of the increasing lipophilicity for the transdermal absorption was proven by carrying out a structure-absorption experiment with six different steroids. In the following publications: Int. J. Pharm. 2001, 217, 1, and J. Chromatography, 49, 631 (1993) it has been shown, that the chromatographical hydrophobicity index (CHI) measured under isocratical conditions is a very good lipophilicity descriptor. We determined the CHI values of the investigated steroids according to these experiments and correlated with the measured stratum corneum/water distribution coefficients The obtained good correlation ($r^2$=0.88) show, that the steroid having a higher CHI index penetrates better across the stratum corneum. According to our measurements the CHI index of E-isomer is higher than that of the Z-isomer, therefore this analysis also prove the transdermal applicability of the E-isomer. The relation between the stratum corneum/water permeability coefficients (logkp) and the CHI indexes is shown in Figure 1.

The invention is illustrated by the following not limiting examples.

**Example 1.**

**d-(17α)-13-Ethyl-17-hydroxy-18,19-dinorpregn-4-ene-20-yne-3-one-(E/Z)-oxime**

34.7 g (0.5 mol) of hydroxylammonium hydrochloride and 34 g (0.41 mol) of sodium acetate are suspended in

500 ml of glacial acetic acid, and after stirring for 1 h 31.2 g (0.1 mol) of d-norgestrel is added under nitrogen. The heterogeneous reaction mixture is stirred till the completion of the reaction and then poured into 3000 ml of water. The precipitated product is filtered off, washed successively with water, 5 % aqueous ammonium hydroxide solution, water and dried below 60 ˚C in vacuum.

The obtained crude product is dissolved in 320 ml of ethanol, clarified with charcoal and after filtering the charcoal the solution is concentrated to a volume of 10 % of the original one. The residue is cooled to 0 ˚C and filtered after 5 h. The solid material is washed ethanol and dried to yield 29.4 g (90 %) of the title compound.

Mp.: 110-130 ˚C (a mixture of geometric isomers).

The ratio of the oxime isomers: E-oxime = 58 %; Z-oxime = 42 %.

## Example 2

### d-(17α)-13-Ethyl-17-hydroxy-18,19-dinorpregn-4-ene-20-yne-3-one-(3E)-oxime

Under nitrogen, to a vigorously stirred suspension of 2.5 g (0.035 mol) of hydroxylammonium hydrochloride, 2 g (0.024 mol) of sodium acetate and 55 ml of 70 % aqueous acetic acid 5 g (0.016 mol) of d-norgestrel is added and stirring is continued for 50 h. Then reaction mixture is poured into 500 ml of water, the precipitated product is filtered off, washed successively with water, 5 % aqueous ammonium hydroxide solution, water and dried below 60 ˚C. The obtained crude product (the ratio of the oxime isomers: E-oxime = 94.5 %; Z-oxime = 5.5 %) is recrystallized from dichloromethane to yield 4.65 g (88.7 %) of the title compound, the pure E-isomer. Mp.: 198-200 ˚C.

## Example 3

### d-(17α)-13-Ethyl-17-hydroxy-18,19-dinorpregn-4-ene-20-yne-3-one-(3E)-oxime

5 g (0.07 mol) of hydroxylammonium hydrochloride and 5.8 g (0.07 mol) of sodium acetate is suspended in 100 ml of glacial acetic acid, the suspension is stirred for 1 h and the formed sodium chloride is filtered off. Under nitrogen, 10 g (0.032 mol) of d-norgestrel is added to the stirred filtrate and stirring is continued till the completion of the reaction. Then 30 ml of water is added to the reaction mixture and stirring is continued for a further 50 h. the reaction mixture is poured into 1000 ml of water, the precipitated product is filtered off, washed according to the method described in example 2 and dried. The crude product is recrystallized from acetonitrile to yield 9.1 g (86.8 %) of the title compound, the pure E-isomer. Mp.: 198-200 ˚C.

## Example 4

### d-(17α)-13-Ethyl-17-hydroxy-18,19-dinorpregn-4-ene-20-yne-3-one-(3E)-oxime

Under nitrogen, to a vigorously stirred suspension of 10 g (0.027 mol) of d-(17α)-13-ethyl-17-hydroxy-18,19-dinorpregn-4-ene-20-yne-3-one-3(E/Z)-oxime [ratio of isomers: E-oxime 58 %, Z-oxime 42 %] and 100 ml of glacial acetic acid 2.5 g (0.035 mol) of hydroxylammonium hydrochloride and 2.9 g (0.035 mol) of sodium acetate in 20 ml of water are added. The reaction mixture is stirred for 50 h, then poured into 1000 ml of water. Further on the method described in example 2 was followed to yield 9.6 g (96 %) of the crude product. The obtained crude product [ratio of isomers: E-oxime 94 %, Z-oxime 6 %] is recrystallized from ethyl acetate according to the method described in example 2 to yield 9.1 g (91 %) of the title compound, the pure E-isomer. Mp.: 197-199 ˚C.

## Example 5

### d-(17α)-13-Ethyl-17-hydroxy-18,19-dinorpregn-4-ene-20-yne-3-one-(3Z)-oxime

A suspension of 43.8 g (0.53 mol) of sodium acetate, 50 g (0.72 mol) of hydroxylammonium hydrochloride and 100 ml of 90 % aqueous acetic acid is vigorously stirred at room temperature for 1 h. The precipitated sodium chloride is filtered off, 100 g (0.32 mol) of d-norgestrel is added to the filtrate under nitrogen and the resulted mixture is stirred for 1.5 h. During this time the temperature of the reaction is allowed to rise to 45 ˚C. The reaction mixture becomes homogeneous, which indicates the completion of the reaction. The reaction mixture is poured into 4000 ml of water, the precipitated product is filtered off, washed successively with water, 5 % aqueous ammonium hydroxide solution, water and dried. The obtained 104 g isomeric mixture of oximes [ratio of isomers: E-oxime 57.4 %, Z-oxime 42.6 %] is vigorously stirred with 20-fold volume of dichloromethane for 30 min, the insoluble material is filtered off and dried below 60 ˚C to yield 45.6 g of product [ratio of isomers: E-oxime 94.4 %, Z-oxime 4.6 %].

The mother liquor obtained after isolating the above product is concentrated to yield 58 g of product [ratio of isomers: Z-oxime 65.5 %, E-oxime 33.2 %]. This is dissolved in 2300 ml (40-fold) of dichloromethane and kept at 0-5 ˚C for 5 h. The precipitated crystalline product is filtered off, washed with dichloromethane and dried to yield 17.6 g of product [ratio of isomers: E-oxime 9 %, Z-oxime 91 %].

The so obtained mother liquor is also concentrated, and the residue 39g is purified by column chromatography using 700 g of silica gel as adsorbent and toluene followed by a more polar mixture of toluene-acetone as eluent. The fractions containing the same isomer are concentrated to yield 3.7 g (isomer purity: 94 %) of E-oxime and 25.2 g (isomer purity: 95 %) of Z-oxime.

The corresponding crystals obtained by crystallization and by column chromatography are combined and recrystallized first from 20-fold volume of acetonitrile, then from 23-fold volume of ethyl acetate to yield 29 g (purity: 99.3 %) of Z-oxime and 38.4 g (purity: 99.7 %) of E-oxime. Mp.: Z-oxime: 206-207 ˚C, mp.: E-oxime: 199-200 ˚C.

**NMR data:**

**Z-oxime:**

**$^1$H NMR {500MHz, DMSO-$d_6$(TMS), δ(ppm)}:** 0.92 (3H,t,-$CH_2$-**$CH_3$**), 1.40 (2H,m,-**$CH_2$-**$CH_3$), 2.05 & 2.24 (2H,m & m, H-2), 3.28 (1H,s,≡CH), 5.23 (1H,s,17-OH), 6.40 (1H,m,H-4), 10.12 (1H,s,=N-OH).

**$^{13}$C NMR {125MHz, DMSO-d6(TMS), δ (ppm)}:** 9.4 (-$CH_2$-$CH_3$), 18.3 (-**$CH_2$**-$CH_3$), 26.9 (C-2), 79.6 (C-17), 89.1 (-C≡), 74.9 (≡CH), 111.6 (C-4), 151-.2 (C-3), 152.0 (C-5).

**E-oxime:**

**$^1$H NMR {500MHz, DMSO-$d_6$(TMS), δ (ppm)}:** 0.92 (3H,t,-$CH_2$-**$CH_3$**), 1.40 (2H,m,-**$CH_2$-**$CH_3$), 1.87 & 2.87 (2H,m & m, H-2), 3.28 (1H,s,≡CH), 5.23 (1H,s,17-OH), 5.78 (1H,m,H-4), 10.38 (1H,s,=N-OH).

**$^{13}$C NMR {125MHz, DMSO-$d_6$(TMS), δ (ppm)}:** 9.4 (-$CH_2$-$CH_3$), 18.3 (-$CH_2$-$CH_3$), 20.6 (C-2), 79.6 (C-17), 89.1 (-C=), 74.9 (≡CH), 118.6 (C-4), 154.3 (C-3), 148.1 (C-5).

## Example 6

### d-(17α)-13-Ethyl-17-hydroxy-18,19-dinorpregn-4-ene-20-yne-3-one-(3E)-oxime

Under nitrogen, to a vigorously stirred solution of 10 g (0.027 mol) of d-(17α)-13-ethyl-17-hydroxy-18,19-dinorpregn-4-ene-20-yne-3-one-(3Z)-oxime and 100 ml of 80 % aqueous acetic acid 2.5 g (0.035 mol) of hydroxylammonium hydrochloride and 2.9 g (0.035 mol) of sodium acetate are added. The reaction mixture is stirred for about 50 h, then processed according to the method described in example 4 to yield 8.5 g (85 %) of the title compound, the pure E-oxime. Mp.: 196-198 ˚C.

## Example 7

### d-(17α)-13-Ethyl-17-hydroxy-18,19-dinorpregn-4-ene-20-yne-3-one-(3E)-oxime

Under nitrogen, to a vigorously stirred solution of 5 g (0.01 mol) of d-(17α)-13-ethyl-1.7= acetoxy-18,19-dinorpregn-4-ene-20-yne-3-one-(3Z)-oxime and 50 ml of methanol 1.7 g (0.04 mol) of lithium hydroxide monohydrate is added at 0-5 ˚C and stirring is continued for 2 h. After completion of the reaction - checked by thin layer chromatography - the reaction mixture is poured into 500 ml of water and the pH of the obtained suspension is adjusted to 7.5-9 with acetic acid. The precipitated product is filtered off, washed with water and dried below 60 ˚C in vacuum. The obtained crude product (4.5 g) is recrystallized from acetonitrile to yield 4 g (90.2 %) of d-(17α)-13-ethyl-17-hydroxy-18,19-dinorpregn-4-ene-20-yne-3-one-(3Z)-oxime.
Mp.: 203-204 ˚C
d-(17α)-13-Ethyl-17-hydroxy-18,19-dinorpregn-4-ene-20-yne-3-one-(3E)-oxime is prepared according to the method described above from 5 g of d-(17α)-13-ethyl-17-acetoxy-18,19-dinorpregn-4-ene-20-yne-3-one-(3E)-oxime. Yield: 4.1 g (92.45 %).
Mp.: 198-200 ˚C.

## Example 8

### d-(17α)-13-Ethyl-17-hydroxy-18,19-dinorpregn-4-ene-20-yne-3-one-(3E)-oxime

Under nitrogen, to a vigorously stirred suspension of 1.25 g (0.017 mol) of hydroxylammonium hydrochloride, 1.45 g (0.017 mol) of sodium acetate and 60 ml of 50 % aqueous acetic acid 2.5 g (0.008 mol) of d-norgestrel is added. After completion of the reaction - checked by thin layer chromatography - the reaction mixture is poured into 500 ml of water. The precipitated product is filtered off, washed successively with water, 5 % aqueous ammonium hydroxide

solution, water and dried below 60 ˚C. The crude product is recrystallized from dichloromethane to yield 2.27 g (86.7 %) of the title compound. Mp.: 198-200 ˚C.

**Example 9**

**d-(17α)-13-Ethyl-17-hydroxy-18,19-dinorpregn-4-ene-20-yne-3-one-oxime (mixture of isomers)**

To a vigorously stirred suspension of 5.8 g (0.07 mol) of sodium acetate and 80 ml of glacial acetic acid 5 g (0.07 mol) of hydroxylammonium hydrochloride in 22 ml of water is added. Then 10 g (0.032 mol) of d-norgestrel is added to the reaction mixture under nitrogen and stirring is continued until completion of the reaction. After completion of the reaction - checked by thin layer chromatography - the reaction mixture is poured into 800 ml of water. The precipitated product is filtered off, washed successively with water, 5 % aqueous ammonium hydroxide solution, water and dried below 60 ˚C to yield 8.9 g (84.92 %) of the title compound as a 55.88:44.05 mixture of E/Z-isomers.
Mp.: 110-130 ˚C

**Example 10**

**Pharmaceutical composition containing d-(17α)-13-ethyl-17-hydroxy-18,19-dinorpregn-4-ene-20-yne-3-one-(3Z)-oxime and ethynyl-oestradiol as active ingredients in tablet form**

250 mg of Z-isomer of norelgestromine and 35 mg of ethynyl-oestradiol is mixed homogeneously with 75.715 g of lactose, 22.5 g of microcrystalline cellulose, 1 g of colloid silicon dioxide (Aerosil) and 500 mg of magnesium stearate. The so obtained powder mixture is pressed to tablets of 100 mg without granulation. About 1000 pieces of tablets are obtained.

**Example 11**

**Pharmaceutical composition containing d-(17α)-13-ethyl-17-hydroxy-18,19-dinorpregn-4-ene-20-yne-3-one-(3Z)-oxime and ethynyl-oestradiol as active ingredients in tablet form**

250 mg of Z-isomer of norelgestromine and 35 mg of ethynyl-oestradiol are dissolved in 10 ml of ethanol and the so obtained mixture is sprayed on a homogeneous mixture of 75.715 g of lactose and 20.5 g of cornstarch. Ethanol is removed from the mixture by fluidization drying. The obtained powder mixture containing the active ingredients is granulated with an aqueous solution of 2 g of polyvinylpyrrolidone (PVP) in a fluidization equipment, then dried. 1 g of colloid silicon dioxide and 0.5 g of magnesium stearate are homogenated to the granulated material, and pressed to tablets of 100 mg. About 1000 pieces of tablets are obtained.

**Example 12**

**Pharmaceutical composition containing d-(17α)-13-ethyl-17-hydroxy-18,19-dinorpregn-4-ene-20-yne-3-one-(3Z)-oxime and ethynyl-oestradiol as active ingredients in tablet form**

250 mg of Z-isomer of norelgestromine, 35 mg of ethynyl-oestradiol and 2 g of polyvinylpyrrolidone (PVP) are dissolved in 10 ml of ethanol, and the so obtained mixture is sprayed on a homogeneous mixture of 75.715 g of lactose and 20.5 g of cornstarch in a high shear mixer. The mixture is granulated, and the ethanol is removed in a microwave vacuum drier. 1 g of colloid silicon dioxide and 0.5 g of magnesium stearate are homogenated to the granulated material, and pressed to tablets of 100 mg. About 1000 pieces of tablets are obtained.

**Example 13**

**Pharmaceutical composition containing d-(17α)-13-ethyl-17-hydroxy-18,19-dinorpregn-4-ene-20-yne-3-one-(3E)-oxime and ethynyl-oestradiol as active ingredients in transdermal plaster form**

One piece of matrix type transdermal plaster of 3 layers contains 6.0 mg of E-isomer of norelgestromine and 0.75 mg of ethynyl-oestradiol.
For every plaster unit 6.0 mg of E-isomer of norelgestromine, 0.75 mg of ethynyl-oestradiol, 25 mg of polyvinylpyrrolidone, 20 mg of lauryl lactate (absorption promoting agent) and 248 mg of polyisobutylene are dispersed in a 8:1:1 mixture of hexane/ethyl acetate/ethanol at room temperature for 45 min. The so obtained dispersion is poured

onto the external membrane of the plaster and dried at 70 ˚C for about 45 min. A protective membrane is layered on the surface of the dried matrix.

**Example 14**

**Pharmaceutical composition containing d-(17α)-13-ethyl-17-hydroxy-18,19-dinorpregn-4-ene-20-yne-3-one-(3E)-oxime and ethynyl-oestradiol as active ingredients in transdermal plaster form**

One piece of matrix type transdermal plaster of 3 layers contains 6.0 mg of E-isomer of norelgestromine and 0.75 mg of ethynyl-oestradiol.

For every plaster unit 261 mg of polydimethylsiloxane and 17 mg of polyvinylpyrrolidone is homogenated at room temperature. 15 mg of methyl laureate, 6.0 mg of E-isomer of norelgestromine and 0.75 mg of ethynyl-oestradiol are added to the mixture and it is dispersed with 350 ml of ethanol at room temperature for 45 min. The so obtained dispersion is poured onto the external membrane of the plaster and dried at 70 ˚C for about 45 min. A protective membrane is layered on the surface of the dried matrix.

## Claims

1. Plaster type pharmaceutical compositions, **characterized by** containing the pure d-(17α)-13-ethyl-17-hydroxy-18,19-dinorpregn-4-ene-20-yne-3-one-(3E)-oxime of formula (IA)

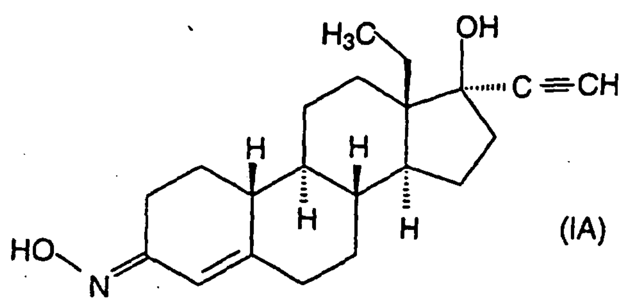

(IA)

as gestagen active ingredient.

2. Orally applicable pharmaceutical compositions, **characterized by** containing the pure d-(17α)-13-ethyl-17-hydroxy-18,19-dinorpregn-4-ene-20-yne-3-one-(3Z)-oxime of formula (IB)

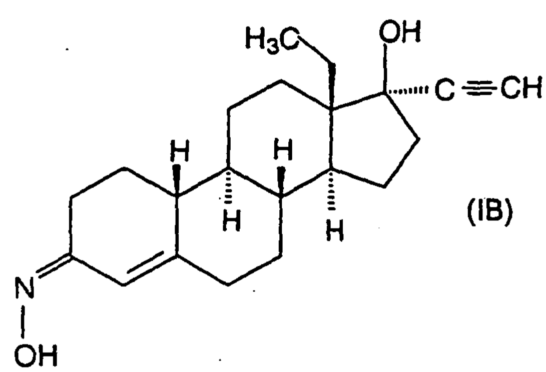

(IB)

as gestagen active ingredient.

3. Process for the synthesis of E/Z-isomeric mixture of norelgestromine, as well as the pure d-(17α)-13-ethyl-17-hydroxy-18,19-dinorpregn-4-ene-20-yne-3-one-(3E)-oxime of formula (IA) and the pure d-(17α)-13-ethyl-17-hydroxy-18,19-dinorpregn-4-ene-20-yne-3-one-(3Z)-oxime of formula (IB), **characterized by**

a) reacting 1 mol of d-norgestrel with 1.2-5 mol equivalent of hydroxylammonium acetate or with a hydroxylammonium salt and not more than one equivalent amount of the latter of alkali metal acetate in acetic acid containing not more than 50 mass percent of water, at 15-50 °C for 15-45 min and the obtained reaction mixture containing the isomeric mixture of norelgestromine

α) is diluted with about a 10-fold volume of water and the precipitated isomeric mixture is isolated to give an E/Z mixture of isomers in a ratio of about 56:44-64:36, or in given case
β) after addition of about 10-25 volume percent of water it is stirred at 10-30 °C for 24-72 h, in given case water is added to the reaction mixture and the precipitated product is isolated to give the (3E)-oxime isomer of formula (IA), or in given case
γ) after addition of about 10-fold volume of water the precipitated isomeric mixture is isolated and stirred in dichloromethane, the insoluble (3E)-oxime isomer of formula (IA) is filtered off, the filtrate is purified by column chromatography using silica gel as adsorbent and a mixture of apolar-polar solvents as fluent to give the (3Z)-oxime of formula (IB), or

b) a mixture of E/Z isomers of norelgestromine of any ratio

α) is stirred with hydroxylammonium acetate or with a hydroxylammonium salt and not more than one equivalent amount of the latter of alkali metal acetate in acetic acid containing not more than 50 mass percent of water, at 15-30 °C for 24-72 h and in given case after addition of more water the product is isolated to give the (3E)-oxime isomer of the formula (IA), or
β) is stirred in dichloromethane; the insoluble (3E)-oxime isomer of formula (IA) is faltered off, the filtrate is purified by column chromatography using silica gel as adsorbent and a mixture of apolar-polar solvents as eluent to give the (3Z)-oxime of formula (IB), or

c) the acetate group at position 17 of the 3E- or 3Z-isomer of norgestimate is hydrolyzed in alcoholic solution with equivalent amount of alkali metal hydroxide at 5-30 °C and the obtained product having the same configuration as the starting material is isolated to give the (3E)-oxime isomer of the formula (IA) or the (3Z)-oxime isomer of formula (IB)

and the isomers of formula (IA) and (IB) obtained according to processes a)-c) are purified by crystallization.

4. Process c) according to claim 3, **characterized by** carrying out the hydrolysis with lithium hydroxide in methanol.

## Patentansprüche

1. Pharmazeutische Zusammensetzung vom Pflaster-Typ, **dadurch gekennzeichnet, dass** sie als Gestagen-Wirkstoff das reine d-(17α)-13-ethyl-17-hydroxy-18,19-dinorpregn-4-en-20-in-3-on-(3E)-oxim der Formel (IA):

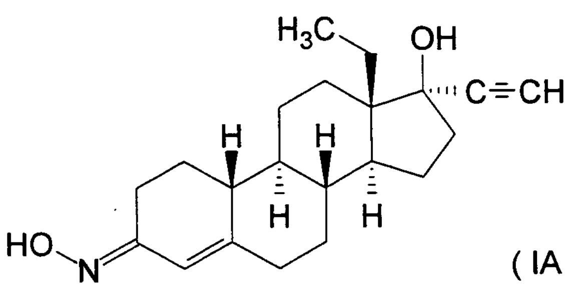

(IA)

enthält.

2. Oral verabreichbare pharmazeutische Zusammensetzung, **dadurch gekennzeichnet, dass** sie als Gestagen-Wirkstoff das reine d-(17α)-13-ethyl-17-hydroxy-18,19-dinorpregn-4-en-20-in-3-on-(3Z)-oxim der Formel (IB) :

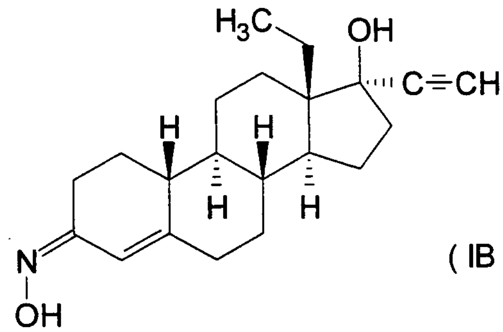

( IB )

enthält.

**3.** Verfahren zur Synthese der E/Z-Isomerenmischung von Norelgestromin sowie des reinen d-(17α)-13-ethyl-17-hydroxy-18,19-dinorpregn-4-en-20-in-3-on-(3E)-oxims der Formel (IA) und des reinen d-(17α)-13-ethyl-17-hydroxy-18,19-dinorpregn-4-en-20-in-3-on-(3Z)-oxims der Formel (IB), **gekennzeichnet durch**

(a) Umsetzen von 1 mol d-Norgestrel mit 1,2 bis 5 Moläquivalenten Hydroxylammoniumacetat oder mit einem Hydroxylammoniumsalz und einer zum letzteren nicht mehr als einfach äquivalenten Menge an Alkalimetallacetat in Essigsäure, die nicht mehr als 50 Masse-% Wasser enthält, bei 15 bis 50°C für 15 bis 45 Minuten; und die erhaltene Reaktionsmischung, die die Isomerenmischung von Norelgestromin enthält,

(α) wird mit dem etwa 10-fachen Volumen an Wasser verdünnt, und die gefällte Isomerenmischung wird isoliert, um eine E/Z-Mischung der Isomeren in einem Verhältnis von etwa 56:44 bis 64:36 zu erhalten, oder gegebenenfalls
(β) nach dem Hinzufügen von etwa 10 bis 25 Vol.% Wasser wird sie bei 10 bis 30°C für 24 bis 72 Stunden gerührt, gegebenenfalls wird Wasser zur Reaktionsmischung hinzugefügt und das gefällte Produkt wird isoliert, um das (3E)-Oximisomer der Formel (IA) zu ergeben, oder gegebenenfalls
(γ) nach dem Hinzufügen des etwa 10-fachen Volumens an Wasser wird die gefällte Isomerenmischung isoliert und in Dichlormethan gerührt, das unlösliche (3E)-Oximisomer der Formel (IA) wird abfiltriert, das Filtrat mittels Säulenchromatografie unter Verwendung von Kieselgel als Adsorbens und einer Mischung von apolar-polaren Lösungsmitteln als Eluent gereinigt, um das (3Z)-Oxim der Formel (IB) zu ergeben, oder

(b) eine Mischung von E/Z-Isomeren von Norelgestromin in beliebigem Verhältnis

(α) wird mit Hydroxylammoniumacetat oder mit einem Hydroxylammoniumsalz und einer zum letzteren nicht mehr als einfach äquivalenten Menge an Alkalimetallacetat in Essigsäure, die nicht mehr als 50 Gew.% an Wasser enthält, bei 15 bis 30°C für 24 bis 72 Stunden gerührt und gegebenenfalls nach Hinzufügen von weiterem Wasser wird das Produkt isoliert, um das (3E)-Oximisomer der Formel (IA) zu ergeben, oder
(β) wird in Dichlormethan gerührt, das unlösliche (3E)-Oximisomer der Formel (IA) wird abfiltriert, das Filtrat wird mittels Säulenchromatografie unter Verwendung von Kieselgel als Adsorbens und einer Mischung aus apolar-polaren Lösungsmitteln als Eluent gereinigt, um das (3Z)-Oxim der Formel (IB) zu ergeben, oder

(c) die Acetatgruppe an Position 17 des 3E- oder 3Z-Isomers von Norgestimat wird in alkoholischer Lösung mit einer äquivalenten Menge von Alkalimetallhydroxid bei 5 bis 30°C hydrolysiert und das erhaltene Produkt, das die gleiche Konfiguration wie das Ausgangsmaterial hat, wird isoliert, um das (3E)-Oximisomer der Formel (IA) oder das (3Z)-Oximisomer der Formel (IB) zu ergeben,

und die **durch** die Verfahren (a) bis (c) erhaltenen Isomere der Formeln (IA) und (IB) werden **durch** Kristallisation gereinigt.

**4.** Verfahren (c) gemäss Anspruch 3, **dadurch gekennzeichnet, dass** die Hydrolyse mit Lithiumhydroxid in Methanol durchgeführt wird.

## Revendications

1. Compositions pharmaceutiques de type emplâtre, **caractérisées en ce qu'**elles contiennent du d-(17α)-13-éthyl-17-hydroxy-18,19-dinorpregn-4-ène-20-yne-3-one-(3E)-oxime pur de formule (IA)

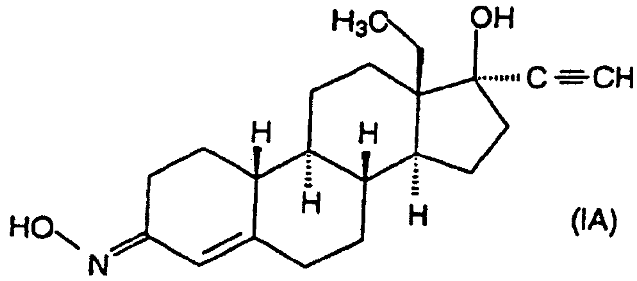

(IA)

comme ingrédient actif gestagène.

2. Compositions pharmaceutiques applicables par voie orale, **caractérisées en ce qu'**elles contiennent du d-(17α)-13-éthyl-17-hydroxy-18,19-dinorpregn-4-ène-20-yne-3-one-(3Z)-oxime pur de formule (IB)

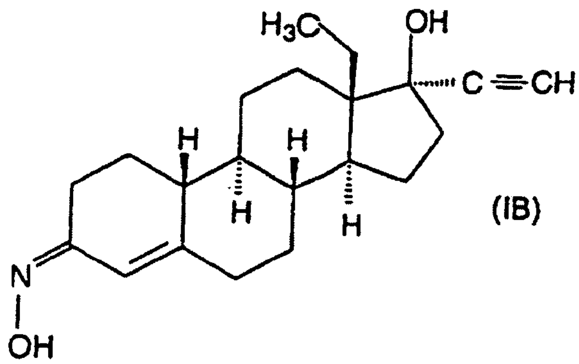

(IB)

comme ingrédient actif gestagène.

3. Procédé de synthèse d'un mélange d'isomères E/Z de norelgestromine, ainsi que du d-(17α)-13-éthyl-17-hydroxy-18,19-dinorpregn-4-ène-20-yne-3-one-(3E)-oxime pur de formule (IA) et du d-(17α)-13-éthyl-17-hydroxy-18,19-dinorpregn-4-ène-20-yne-3-one-(3Z)-oxime pur de formule (IB), **caractérisé par**

   a) la réaction de 1 mol de d-norgestrel avec 1,2 à 5 équivalents molaires d'acétate d'hydroxylammonium ou avec un sel d'hydroxylammonium et pas plus d'une quantité équivalente de ce dernier d'un acétate de métal alcalin dans de l'acide acétique ne contenant pas plus de 50 pour cent en masse d'eau, à 15 à 50 °C pendant 15 à 45 min, et le mélange de réaction obtenu contenant le mélange isomère de norelgestromine

      α) est dilué avec un volume d'eau d'environ 10 fois et le mélange isomère précipité est isolé pour donner un mélange E/Z d'isomères en un rapport d'environ 56 : 44 à 64 : 36, ou dans un cas donné
      β) après l'addition d'environ 10 à 25 pour cent en volume d'eau, il est agité à 10 à 30 °C pendant 24 à 72 h, dans un cas donné de l'eau est ajoutée au mélange de réaction et le produit précipité est isolé pour donner l'isomère du (3E)-oxime de formule (IA), ou dans un cas donné
      γ) après l'addition d'un volume d'eau d'environ 10 fois, le mélange isomère précipité est isolé et agité dans du dichlorométhane, l'isomère du (3E)-oxime insoluble de formule (la) est éliminé par filtration, le filtrat est purifié par chromatographie sur colonne en utilisant du gel de silice comme adsorbant et un mélange de solvants apolaires-polaires comme éluant pour donner le (3Z)-oxime de formule (IB), ou

b) un mélange d'isomères E/Z de norelgestromine de rapport quelconque

α) est agité avec de l'acétate d'hydroxylammonium ou avec un sel d'hydroxylammonium et pas plus d'une quantité équivalente de ce dernier d'un acétate de métal alcalin dans de l'acide acétique ne contenant pas plus de 50 pour cent en masse d'eau, à 15 à 30 °C pendant 24 à 72 h, et dans un cas donné après addition davantage d'eau, le produit est isolé pour donner l'isomère de la (3E)-oxime de formule (IA), ou
β) est agité dans du dichlorométhane, l'isomère de la (3E)-oxime insoluble de formule (IA) est éliminé par filtration, le filtrat est purifié par chromatographie sur colonne en utilisant un gel de silice comme adsorbant et un mélange de solvants apolaires-polaires comme éluant pour donner le (3Z)-oxime de formule (IB), ou

c) le groupe acétate en position 17 de l'isomère 3E ou 3Z de norgestimate est hydrolysé dans une solution alcoolique avec une quantité équivalente d'hydroxyde de métal alcalin à 5 à 30 °C et le produit obtenu ayant la même configuration que la matière de départ est isolé pour donner l'isomère du (3E)-oxime de formule (IA) ou l'isomère du (3Z)-oxime de formule (IB)

et les isomères de formule (IA) et (IB) obtenus selon les procédés a) à c) sont purifiés par cristallisation.

**4.** Procédé c) selon la revendication 3, **caractérisé en ce que** de l'hydrolyse est réalisée avec de l'hydroxyde de lithium dans du méthanol.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 3780073 A
- US 4027019 A
- WO 9640355 A
- HU 165356


### Non-patent literature cited in the description


- *Am. J. Obstet. Gynecol.,* 1992, vol. 166, 1969-77
- *Am. J. Obstet. Gynecol.,* 1990, vol. 163, 2127-31
- *J. Chromatogr.,* 1987, vol. 392, 464-9
- *J. Chromatogr.,* 1980, vol. 191 (1), 145-54
- **Hara.** *Chem. Ind. (London),* 1967, 832
- *Journal of Chromatography,* 1993, vol. 635, 342-345
- *J. Chromatogr.,* 1993, vol. 635, 342-345
- **Siddiqui.** *J. Pharm. Biopharm.,* 1989, vol. 17, 405
- *Int. J. Pharm.,* 2001, vol. 217, 1
- *J. Chromatography,* 1993, vol. 49, 631